(19) Europäisches Patentamt
European Patent Office
Office européen des brevets

(11) **EP 2 509 653 B1**

(12) **EUROPÄISCHE PATENTSCHRIFT**

(45) Veröffentlichungstag und Bekanntmachung des Hinweises auf die Patenterteilung:
**25.06.2014 Patentblatt 2014/26**

(21) Anmeldenummer: **10795935.5**

(22) Anmeldetag: **08.12.2010**

(51) Int Cl.:
*A61M 1/16* *(2006.01)*

(86) Internationale Anmeldenummer:
**PCT/EP2010/007451**

(87) Internationale Veröffentlichungsnummer:
**WO 2011/069645 (16.06.2011 Gazette 2011/24)**

(54) **VORRICHTUNG UND VERFAHREN ZUR ÜBERWACHUNG EINER IM FLÜSSIGKEITSSYSTEM EINER EXTRAKORPORALEN BLUTBEHANDLUNGSVORRICHTUNG ANGEORDNETEN ELEKTRISCH BETRIEBENEN PUMPE**

DEVICE AND METHOD FOR MONITORING AN ELECTRICALLY OPERATED PUMP ARRANGED IN A LIQUID SYSTEM OF AN EXTRACORPOREAL BLOOD TREATMENT DEVICE

DISPOSITIF ET PROCÉDÉ DE SURVEILLANCE D'UNE POMPE À FONCTIONNEMENT ÉLECTRIQUE DISPOSÉE DANS LE SYSTÈME DE LIQUIDE D'UN DISPOSITIF DE TRAITEMENT EXTRACORPOREL DU SANG

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **11.12.2009 DE 102009057858**

(43) Veröffentlichungstag der Anmeldung:
**17.10.2012 Patentblatt 2012/42**

(73) Patentinhaber: **Fresenius Medical Care Deutschland GmbH**
**61352 Bad Homburg v.d.H. (DE)**

(72) Erfinder: **WERNICKE, Ulrich**
**97531 Theres (DE)**

(74) Vertreter: **Oppermann, Frank**
**OANDO Oppermann & Oppermann LLP**
**Washingtonstrasse 75**
**65189 Wiesbaden (DE)**

(56) Entgegenhaltungen:
**DE-A1- 2 838 414     DE-A1-102006 011 346**
**DE-C1- 19 830 928   US-A- 5 629 871**
**US-A- 6 042 784**

## Beschreibung

Die Erfindung betrifft eine Vorrichtung und ein Verfahren zur Überwachung einer im Flüssigkeitssystem, insbesondere Dialysierflüssigkeitssystem, einer extrakorporalen Blutbehandlungsvorrichtung angeordneten elektrisch betriebenen Pumpe, insbesondere einer elektrischen Pumpe zum Fördern von Dialysierflüssigkeit.

Die bekannten Vorrichtungen zur extrakorporalen Blutbehandlung, beispielsweise Vorrichtungen zur Hämodialyse (HD) oder Hämofiltration (HF) sowie Hämodiafiltration (HDF), verfügen über Bilanziersysteme zur exakten Bilanzierung von der einem Dialysator zugeführten frischen Dialysierflüssigkeit und der aus dem Dialysator abgeführten verbrauchten Dialysierflüssigkeit unter Berücksichtigung der über die Membran des Dialysators dem Patienten entzogenen Menge an Flüssigkeit. Die bekannten volumetrischen Bilanziersysteme arbeiten mit einer oder mehreren Bilanzkammern, die jeweils von einer flexiblen Trennwand in zwei Bilanzkammerhälften getrennt sind. Durch wechselweises Befüllen und Entleeren der beiden Kammerhälften können exakt gleiche Volumina von Dialysierflüssigkeit dem Dialysator zugeführt und aus dem Dialysator abgeführt werden.

Zum Fördern der Flüssigkeiten im Flüssigkeitssystem der extrakorporalen Blutbehandlungsvorrichtung finden im Allgemeinen elektrisch betriebene Pumpen Verwendung. Bei den bekannten Bilanziersystemen stellt sich das Problem, die elektrisch betriebenen Pumpen derart anzusteuern, dass die Bilanzkammerhälften wechselweise befüllt werden. Die Dialysierflüssigkeitspumpe muss dann gestoppt werden, wenn die frische Dialysierflüssigkeit in der einen Bilanzkammerhälfte die verbrauchte Dialysierflüssigkeit aus der anderen Bilanzkammerhälfte verdrängt hat. Daraufhin wird die Bilanzkammerhälfte, die zuvor verbrauchte Dialysierflüssigkeit enthalten hat, wieder mit frischer Dialysierflüssigkeit und die Bilanzkammerhälfte, die zuvor frische Dialysierflüssigkeit enthalten hat, wieder mit verbrauchter Dialysierflüssigkeit befüllt.

Dieser Vorgang wird nachfolgend als Umschalten der Bilanzkammer bezeichnet. Zum Umschalten der Bilanzkammer sind bei den bekannten Bilanziereinrichtungen Ventile in den zu den Bilanzkammerhälften führenden Zuführleitungen und den von den Bilanzkammerhälften abgehenden Abführleitungen angeordnet. Ein derartiges Bilanziersystem ist beispielsweise aus der US 6 042 784 bekannt.

Wenn die bewegliche Trennwand in der Bilanzkammer beim Befüllen der einen Bilanzkammerhälfte und Entleeren der anderen Bilanzkammerhälfte ihre Endlage erreicht hat, steigt der Pumpenstrom der elektrisch betriebenen Pumpe zum Fördern der Dialysierflüssigkeit an. Es sind Bilanziereinrichtungen bekannt, bei denen der zeitliche Verlauf des Pumpenstroms zur Ansteuerung der elektrisch betriebenen Pumpe sowie der Ventile im Dialysierflüssigkeitssystem überwacht wird. Wenn der Pumpenstrom einen vorgegebenen Grenzwert überschreitet, wird ein Steuersignal (Trigger) zum Stoppen der Dialysierflüssigkeitspumpe und Umschalten der Ventile erzeugt. Eine derartige Bilanziervorrichtung ist z.B. aus der DE 19 830 928 bekannt.

Die Überwachung des Pumpenstroms hat sich in der Praxis bewährt. Nachteilig ist jedoch, dass der Pumpenstrom von verschiedenen Faktoren abhängig ist, die sich ändern können. Beispielsweise unterliegen die Pumpen, insbesondere die verwendeten Zahnradpumpen, einem dauernden Verschleiß, wobei sich mit zunehmendem Alter der Pumpe der Pumpenstrom ändert. Der Pumpenstrom ist aber auch von der eingestellten Förderrate der Pumpe abhängig. Dabei zeigt sich, dass die Änderung des Pumpenstroms aufgrund veränderter Flussraten wesentlich größer als der Anstieg des Pumpenstroms ist, wenn die bewegliche Trennwand in der Bilanzkammer die Endlage erreicht hat.

Der Erfindung liegt die Aufgabe zugrunde, eine Vorrichtung zur Überwachung einer im Flüssigkeitssystem einer extrakorporalen Blutbehandlungsvorrichtung angeordneten elektrisch betriebenen Pumpe zu schaffen, die mit hoher Zuverlässigkeit und hoher Sicherheit den periodisch auftretenden Stromanstieg erkennt.

Darüber hinaus liegt der Erfindung die Aufgabe zugrunde, ein Verfahren zur Überwachung einer im Flüssigkeitssystem einer extrakorporalen Blutbehandlungsvorrichtung angeordneten elektrisch betriebenen Pumpe bereit zu stellen, mit dem mit hoher Zuverlässigkeit und hoher Sicherheit ein periodisch auftretender Stromanstieg des Pumpenstroms erkannt werden kann.

Eine weitere Aufgabe der Erfindung ist, eine extrakorporale Blutbehandlungsvorrichtung mit einer derartigen Vorrichtung zur Überwachung der im Flüssigkeitssystem der Blutbehandlungsvorrichtung angeordneten elektrisch betriebenen Pumpe zu schaffen.

Die Lösung dieser Aufgaben erfolgt erfindungsgemäß mit den Merkmalen der Patentansprüche 1, 9, 10 und 11. Vorteilhafte Ausführungsformen der Erfindung sind Gegenstand der Unteransprüche.

Die erfindungsgemäße Vorrichtung zur Überwachung zeichnet sich dadurch aus, dass die Auswerteinheit über Mittel zum fortlaufenden Bestimmen des vorgegebenen Grenzwerts verfügt, mit dem der Pumpenstrom oder eine mit dem Pumpenstrom korrelierende Größe verglichen wird. Unter einer fortlaufenden Bestimmung des vorgegebenen Grenzwertes wird sowohl eine kontinuierliche als auch diskontinuierliche Bestimmung des Grenzwerts verstanden. Allein entscheidend ist, dass der vorgegebene Grenzwert während der Überwachung laufend angepasst wird.

Die Mittel zum Vergleichen des Pumpenstroms oder der mit dem Pumpenstrom korrelierenden Größe sind derart ausgebildet, dass der Pumpenstrom oder die mit dem Pumpenstrom korrelierende Größe mit dem fortlaufend berechneten Grenzwert zur Erkennung eines periodisch auftretenden Anstiegs des Pumpenstroms oder der mit dem Pumpenstrom korrelierenden Größe verglichen wird. Da der vorgegebene Grenzwert laufend angepasst wird, hat beispielsweise die Alterung der Pumpe oder deren Förderrate keinen Einfluss auf die Erzeugung des Steuersignals. Eine Nachjustierung oder Kalibrierung der Pumpe ist daher nicht erforderlich. Somit wird das Steuersignal mit hoher Sicherheit und großer Zuverlässigkeit erzeugt.

Die mit dem Pumpenstrom korrelierende Größe kann beispielsweise die in einem Messwiderstand abfallende Spannung sein, die sich nach dem Ohmschen Gesetz aus dem Produkt von Strom und Widerstand berechnet.

Bei einer bevorzugten Ausführungsform sind die Mittel zum fortlaufenden Bestimmen des vorgegebenen Grenzwerts derart ausgebildet, dass der vorgegebene Grenzwert auf der Grundlage von zwei charakteristischen Werten ermittelt wird, die fortlaufend bestimmt werden. Der erste Wert ist für den Pumpenstrom oder die mit dem Pumpenstrom korrelierende Größe vor dem periodisch auftretenden Anstieg charakteristisch, während der zweite Wert für den Pumpenstrom oder die mit dem Pumpenstrom korrelierende Größe während des periodisch auftretenden Anstiegs des Pumpenstroms oder der mit dem Pumpenstrom korrelierenden Größe charakteristisch ist.

Bei einer weiteren bevorzugten Ausführungsform ist der erste Wert der Minimalwert des Pumpenstroms oder der mit dem Pumpenstrom korrelierenden Größe vor dem periodisch auftretenden Anstieg, während der zweite Wert der Maximalwert des Pumpenstroms oder der mit dem Pumpenstrom korrelierenden Größe während des periodisch auftretenden Anstiegs ist. Aus dem Minimalwert und dem Maximalwert wird dann der vorgegebene Grenzwert bestimmt.

Der vorgegebene Grenzwert wird bei einer besonders bevorzugten Ausführungsform aus dem Minimalwert und dem Maximalwert berechnet. Bei einer besonders bevorzugten Ausführungsform ist der vorgegebene Grenzwert die Summe des Minimalwertes und der Hälfte der Differenz zwischen Maximal- und Minimalwert. Folglich liegt der vorgegebene Grenzwert zwischen Minimal- und Maximalwert auf der Flanke des Stromanstiegs.

Zur laufenden Anpassung der beiden Werte für die Berechnung des vorgegebenen Grenzwerts wird der Pumpenstrom oder die mit dem Pumpenstrom korrelierende Größe vorzugsweise kontinuierlich oder diskontinuierlich überwacht, wobei der momentan gemessene Wert für den Pumpenstrom oder die mit dem Pumpenstrom korrelierende Größe mit dem zuvor ermittelten Minimalwert bzw. mit dem zuvor ermittelten Maximalwert für den Pumpenstrom oder die mit dem Pumpenstrom korrelierende Größe verglichen wird. Wenn der momentan gemessene Wert kleiner bzw. größer als der zuvor gemessene Wert ist, wird der momentan gemessene Wert als neuer Minimalwert bzw. Maximalwert angenommen.

Eine weitere besonders bevorzugte Ausführungsform sieht vor, dass bei der Erkennung eines periodisch auftretenden Anstiegs des Pumpenstroms oder der mit dem Pumpenstrom korrelierenden Größe ein Zeitglied mit einem vorgegebenen Zeitintervall gestartet wird, wobei innerhalb des vorgegebenen Zeitintervalls die Erzeugung des Steuersignals unterdrückt wird. Dies hat den Vorteil, dass in der Praxis auftretende Störimpulse auf dem Messsignal, die auf die Umschaltung der Bilanzkammer zurückzuführen sind, keinen Einfluss auf die Erzeugung des Steuersignals haben können. Von Vorteil ist auch, wenn während des vorgegebenen Zeitintervalls die Bestimmung des ersten und zweiten Wertes, insbesondere des Minimal- und Maximalwertes für den Pumpenstrom oder der mit dem Pumpenstrom korrelierenden Größe, unterbrochen wird. Dadurch wird vermieden, dass Störimpulse auf dem Messsignal zu einer falschen Anpassung des vorgegebenen Grenzwertes führen können.

Im Folgenden wird ein Ausführungsbeispiel der Erfindung unter Bezugnahme auf die Zeichnungen näher erläutert.

Es zeigen:

Fig. 1    eine Vorrichtung zur extrakorporalen Blutbehandlung mit einer Vorrichtung zur Überwachung der im Flüssigkeitssystem der Blutbehandlungsvorrichtung angeordneten Dialysierflüssigkeitspumpe in stark vereinfachter schematischer Darstellung und

Fig. 2    den zeitlichen Verlauf einer mit dem Pumpenstrom der Dialysierflüssigkeitspumpe der Vorrichtung zur extrakorporalen Blutbehandlung von Fig. 1 korrelierenden Größe.

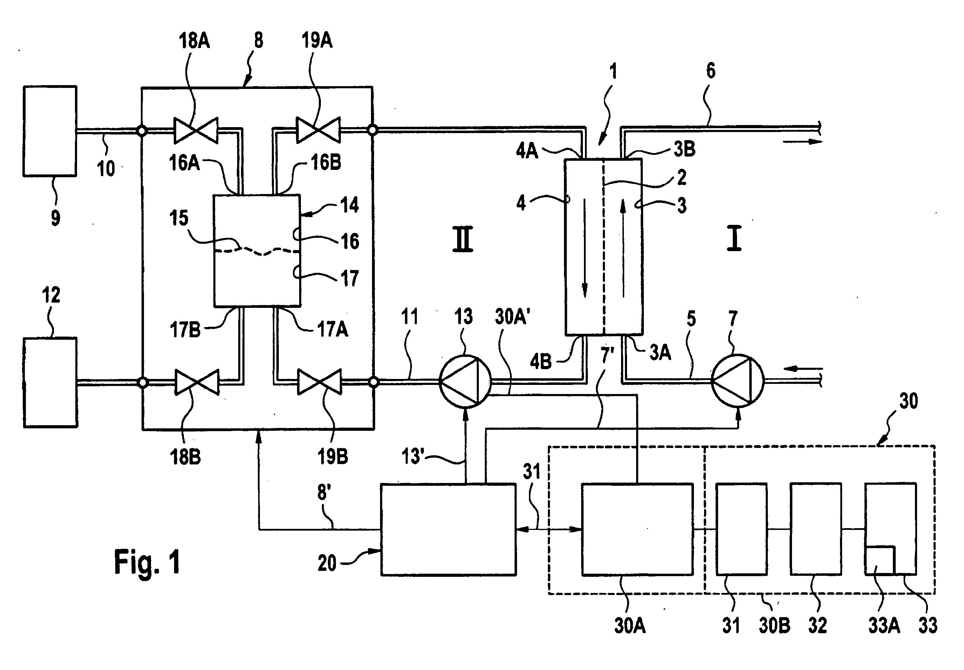

Fig. 1 zeigt die wesentlichen Komponenten der erfindungsgemäßen Vorrichtung zur extrakorporalen Blutbehandlung in stark vereinfachter schematischer Darstellung, die über die erfindungsgemäße Vorrichtung zur Überwachung der im Dialysierflüssigkeitssystem der Blutbehandlungsvorrichtung angeordneten Pumpe zum Fördern von Dialysierflüssigkeit verfügt.

Die extrakorporale Blutbehandlungsvorrichtung weist einen Dialysator oder Filter 1 auf, der durch eine semipermeable Membran 2 in eine Blutkammer 3 und eine Dialysierflüssigkeitskammer 4 unterteilt ist. Von dem Patienten führt eine Blutzuführleitung 5 zu dem Einlass 3A der Blutkammer 3, während von dem Auslass 3B der Blutkammer 3 des Dialysators 1 eine Blutabführleitung 6 zu dem Patienten führt. In die Blutzuführleitung 5 ist eine Blutpumpe 7 zum Fördern des Bluts im extrakorporalen Blutkreislauf I geschaltet.

Das Dialysierflüssigkeitssystem II der Blutbehandlungsvorrichtung umfasst eine Bilanziereinrichtung 8. Da Bilanziereinrichtungen und Blutbehandlungsvorrichtungen dem Fachmann allgemein bekannt sind, wird die Bilanziereinrichtung 8 nur in stark vereinfachter schematischer Darstellung gezeigt. Die Bilanziereinrichtung bilanziert frische gegen verbrauchte Dialysierflüssigkeit. Die frische Dialysierflüssigkeit wird in einer Dialysierflüssigkeitsquelle 9 bereitgestellt. Von der Dialysierflüssigkeitsquelle 9 führt eine Dialysierflüssigkeitszuführleitung 10 zu dem Einlass 4A der Dialysierflüssigkeitskammer 4, während von dem Auslass 4B der Dialysierflüssigkeitskammer 4 eine Dialysierflüssigkeitsabführleitung 11 zu einem Abfluss 12 führt.

Die Bilanziereinrichtung 8 ist in die Dialysierflüssigkeitszuführ- und -abführleitung 10, 11 geschaltet. In dem Abschnitt der Dialysierflüssigkeitsabführleitung 11 zwischen Dialysator 1 und Bilanziereinrichtung 8 befindet sich eine Dialysierflüssigkeitspumpe 13 zum Fördern der Dialysierflüssigkeit. Die Dialysierflüssigkeitspumpe 13 ist eine elektrisch betriebene Pumpe, insbesondere eine Zahnradpumpe.

Die Bilanziereinrichtung 8 weist vorzugsweise zwei Bilanzkammern auf, von denen nur eine Bilanzkammer 14 schematisch dargestellt ist. Die Bilanzkammer 14 ist durch eine bewegliche Trennwand 15 in eine erste und zweite Bilanzkammerhälfte 16, 17 unterteilt. Die Bilanzkammerhälfte 16 bzw. 17 weist einen Zulauf 16A bzw. 17A und einen Ablauf 16B bzw. 17B auf. In den Zulauf bzw. Ablauf jeder Bilanzkammerhälfte 16,17 ist ein Absperrorgan 18A, 18B bzw. 19A, 19B geschaltet, um den Zu- und Ablauf absperren bzw. öffnen zu können. Die Absperrorgane sind elektromagnetisch betätigbare Ventile, die geöffnet bzw. geschlossen werden, um eine Bilanzkammerhälfte mit frischer bzw. verbrauchter Dialysierflüssigkeit zu befüllen und die andere Bilanzkammerhälfte mit verbrauchter bzw. frischer Dialysierflüssigkeit zu entleeren. Mit dem Öffnen bzw. Schließen der jeweiligen Absperrorgane muss die Dialysierflüssigkeitspumpe 13 gestoppt bzw. gestartet werden. Die Dialysierflüssigkeitspumpe muss gestoppt werden, wenn die eine Bilanzkammerhälfte vollständig befüllt ist und die andere Bilanzkammerhälfte vollständig entleert ist. Der Wechsel zwischen dem Befüllen und Entleeren der Bilanzkammerhälften in den einzelnen Bilanzkammertakten wird nachfolgend als Umschalten der Bilanzkammer 14 bezeichnet.

Die oben beschriebene Bilanziereinrichtung ist dem Fachmann allgemein bekannt. Eine derartige Bilanziereinrichtung ist beispielsweise in der DE 28 38 414 und der US 6 042 784 beschrieben. Bilanziereinrichtungen können in den bekannten extrakorporalen Blutbehandlungsvorrichtungen nicht nur zum Bilanzieren von frischer gegen verbrauchte Dialysierflüssigkeit, sondern auch zum Bilanzieren von frischer Dialysierflüssigkeit gegen Substitutionsflüssigkeit eingesetzt werden.

Die Blutbehandlungsvorrichtung verfügt weiterhin über eine zentrale Steuereinheit 20, die über eine Steuerleitung 7' mit der Blutpumpe 7 und eine Steuerleitung 8' mit der Bilanziereinrichtung 8 und eine Steuerleitung 13' mit der Dialysierflüssigkeitspumpe 13 verbunden ist. Die zentrale Steuereinheit 20 steuert die Absperrorgane 18A, 18B bzw. 19A, 19B der Bilanziereinrichtung 8 und die Dialysierflüssigkeitspumpe 13 an, so dass frische und verbrauchte Dialysierflüssigkeit bilanziert werden, während die Dialysierflüssigkeit durch die Dialysierflüssigkeitskammer 4 des Dialysators 1 strömt.

Die Vorrichtung 30 zur Überwachung der im Dialysierflüssigkeitssystem II angeordneten Dialysierflüssigkeitspumpe 13 wird nachfolgend beschrieben. Anstelle der Dialysierflüssigkeitspumpe kann aber auch eine elektrisch betriebene Substituatpumpe überwacht werden, wenn Dialysierflüssigkeit gegen Substitutionsflüssigkeit bilanziert werden sollte. Die Überwachungsvorrichtung 30 kommuniziert über eine Datenleitung 31 mit der zentralen Steuer- und Recheneinheit 20. Die Überwachungsvorrichtung 30 kann aber auch Bestandteil der Steuereinheit 20 sein.

Die Überwachungsvorrichtung 30 erzeugt ein Steuersignal, das die zentrale Steuer- und Recheneinheit 20 über die Datenleitung 31 empfängt. Wenn die Steuereinheit 20 das Steuersignal empfängt, steuert die Steuereinheit die Bilanziereinrichtung 8, die Dialysierflüssigkeitspumpe 13 und die Absperrorgane 18A, 18B bzw. 19A, 19B derart an, dass die Bilanzkammer 14 umgeschaltet wird, wobei die Dialysierflüssigkeitspumpe 13 angehalten wird.

Die Überwachungsvorrichtung 30 verfügt über eine Messeinheit 30A, die über eine Messleitung 30A' mit der Dialysierflüssigkeitspumpe 13 verbunden ist. Die Messeinheit 30A misst eine an einem nicht dargestellten Messwiderstand abfallende Spannung $U_{mess}$, die nach dem Ohmschen Gesetzt dem Pumpenstrom I der Dialysierflüssigkeitspumpe 13 proportional ist.

Darüber hinaus verfügt die Überwachungsvorrichtung 30 über eine Auswerteinheit 30B zum Auswerten des zeitlichen Verlaufs der gemessenen Spannung $U_{mess}$. Die Auswerteinheit 30B weist Mittel 31 zum Vergleichen der gemessenen Spannung $U_{mess}$ mit einem vorgegebenen Grenzwert $U_{trigger}$ und Mittel 32 zum Erzeugen des Steuersignals auf, wenn die gemessene Spannung $U_{mess}$ größer als der vorgegebene Grenzwert $U_{trigger}$ ist. Weiterhin weist die Auswerteinheit 30B Mittel 33 zum fortlaufenden Bestimmen des vorgegebenen Grenzwerts $U_{trigger}$ auf, die nachfolgend beschrieben werden.

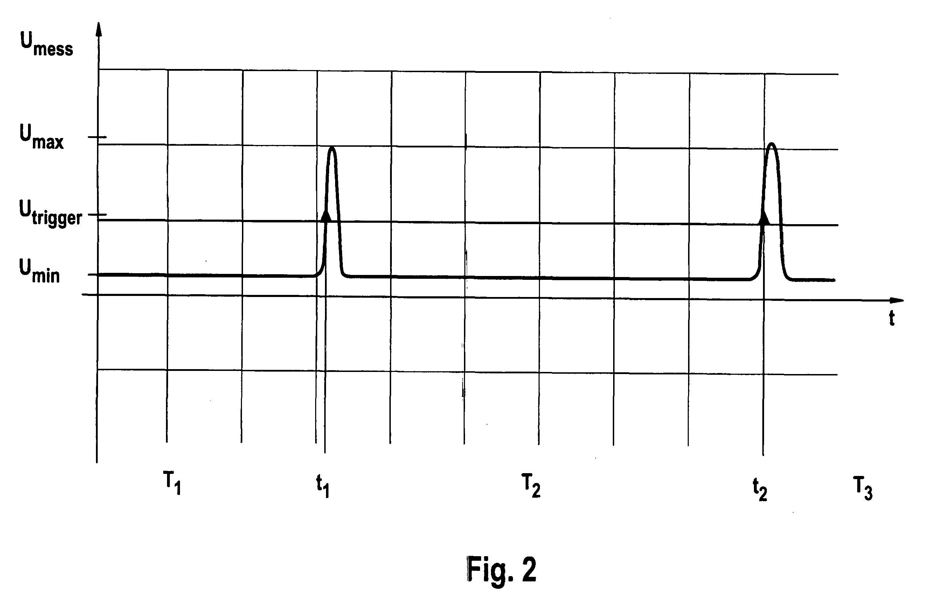

Fig. 2 zeigt den zeitlichen Verlauf der mit der Messeinheit 30A gemessenen Spannung $U_{mess}$. In Fig. 2 ist zu erkennen, dass die Spannung $U_{mess}$ zu den Zeitpunkten $t_1$, $t_2$,... $t_n$ ansteigt, wenn die bewegliche Trennwand 15 der Bilanzkammer 14 ihre Endposition erreicht hat, d.h. beispielsweise die Bilanzkammerhälfte 16 vollständig gefüllt und die Bilanzkammerhälfte 17 vollständig entleert ist. Zu diesem Zeitpunkt muss die Dialysierflüssigkeitspumpe 13 zum Umschalten der Bilanzkammer angehalten werden. Dies ist der Zeitpunkt, zu dem das Steuersignal erzeugt werden soll. Fig. 2 zeigt drei aufeinanderfolgende Bilanzkammertakte $T_1$, $T_2$, $T_3$.

Zur Erkennung des in Fig. 2 mit Pfeilen gekennzeichneten Stromanstiegs, d.h. der ansteigenden Flanke des Stromanstiegs, wird die mit der Messeinheit 30A gemessene Spannung $U_{mess}$ mit einem vorgegebenen Grenzwert $U_{trigger}$ verglichen. Wenn die gemessene Spannung größer als der vorgegebene Grenzwert ist, wird die positive Flanke des Stromanstiegs erkannt.

Der vorgegebene Grenzwert $U_{trigger}$ wird aus der gemessenen Spannung $U_{min}$ vor Auftreten des Stromanstiegs und der gemessenen Spannung $U_{max}$ während des Stromanstiegs nach der folgenden Gleichung berechnet.

$$U_{trigger} = U_{min} + ((U_{max} - U_{min})/2) \qquad (1)$$

Dabei ist $U_{min}$ die minimale Spannung, d.h. die Spannung vor dem Anschlag der flexiblen Trennwand an die Wandung der Bilanzkammer und $U_{max}$ die maximale Spannung beim Anschlag der Trennwand an die Wandung der Bilanzkammer.

Zur Initialisierung werden zunächst zwei Wert für $U_{min}$ und $U_{max}$ ermittelt. Hierzu wird die Spannung laufend gemessen. Wenn die momentan gemessene Spannung $U_{mess}$ größer als die zuvor gemessene Spannung ist, wird die momentan gemessene Spannung als der Maximalwert $U_{max}$ der Spannung angenommen, während die momentan gemessene Spannung als der Minimalwert der gemessenen Spannung $U_{min}$ angenommen wird, wenn der momentan gemessene Wert der Spannung kleiner als der zuvor gemessene Wert der Spannung ist. Folglich werden bei der Initialisierung $U_{min}$ und $U_{max}$ laufend aktualisiert. Bei der Initialisierung wird das Auftreten des ersten Stromanstiegs dann erkannt, wenn die Differenz zwischen $U_{max}$ und $U_{min}$ größer als ein erster vorgegebener Grenzwert ist. Für die dann folgenden Bilanzkammertakte wird der vorgegebene Grenzwert laufend nach der Gleichung (1) berechnet, wobei $U_{min}$ und $U_{max}$ laufend angepasst werden.

Die Berechnung des neuen Grenzwertes in einem Bilanzkammertakt erfolgt jeweils auf der Grundlage der zuvor aktualisierten Werte für $U_{min}$ und $U_{max}$. Folglich wird für jeden Bilanzkammertakt der vorgegebene Grenzwert $U_{trigger}$ neu festgelegt, wenn sich die Werte geändert haben. Wenn also die gemessene Spannung bzw. der Pumpenstrom aufgrund der Alterung der Pumpe zunehmen sollte, findet der auf die Alterung der Pumpe zurückzuführende Anstieg des Ruhestroms bei der Berechnung des vorgegebenen Grenzwerts Berücksichtigung. Auch findet ein Stromanstieg des Ruhestroms Berücksichtigung, der auf eine höhere Förderrate der Pumpe zurückzuführen ist. Daher kann die positive Flanke des Stromanstiegs mit hoher Zuverlässigkeit und großer Sicherheit unabhängig von diesen Faktoren erkannt werden.

In der Praxis hat sich gezeigt, dass unmittelbar nach dem Umschalten der Bilanzkammer Störimpulse auf dem Messsignal $U_{mess}$ auftreten können. Diese Störimpulse können zu einer Erzeugung eines Steuersignals führen, wenn der Störimpuls größer als der Grenzwert ist. Auch können die Störimpulse zu der Ermittlung falscher Werte für $U_{max}$ führen.

Die Mittel 3,3 zum fortlaufenden Bestimmen des vorgegebenen Grenzwertes weisen ein Zeitglied 33A auf, das nach der Feststellung eines Stromanstiegs in einem vorausgehenden Bilanzkammertakt ein vorgegebenes Zeitintervall Δt in Gang setzt. Während dieses vorgegebenen Zeitintervalls Δt wird die Erzeugung eines neuen Steuersignals in einem darauf folgenden Bilanzkammertakt unterdrückt. Das vorgegebene Zeitintervall Δt ist derart bemessen, dass nach Ablauf des Zeitintervalls Störimpulse nicht mehr zu erwarten sind. Das Zeitintervall muss auf jeden Fall kleiner als der Zeitraum zwischen zwei Bilanzkammertakten sein, da ansonsten das Steuersignal für die nächste Umschaltung der Bilanzkammer nicht erzeugt werden könnte.

Während des vorgegebenen Zeitintervalls Δt wird vorzugsweise nicht nur die Erzeugung des Steuersignals unterbrochen, sondern auch die Festlegung neuer Werte für $U_{min}$ und $U_{max}$, so dass die Werte für $U_{min}$ und $U_{max}$ nur dann aktualisiert werden, wenn Störimpulse auf den Messsignal nicht mehr zu erwarten sind.

**Patentansprüche**

1. Vorrichtung zur Überwachung einer im Flüssigkeitssystem einer extrakorporalen Blutbehandlungsvorrichtung angeordneten elektrisch betriebenen Pumpe mit
   einer Messeinheit (30A) zum Messen des Pumpenstroms oder einer mit dem Pumpenstrom korrelierenden Größe,
   einer Auswerteinheit (30B) zum Auswerten des zeitlichen Verlaufs des Pumpenstroms oder der mit dem Pumpenstrom korrelierenden Größe, die aufweist:

   Mittel (31) zum Vergleichen des Pumpenstroms oder der mit dem Pumpenstrom korrelierenden Größe mit einem vorgegebenen Grenzwert,
   Mittel (32) zum Erzeugen eines Steuersignals, wenn der Pumpenstrom oder die mit dem Pumpenstrom korrelierende Größe größer als der vorgegebene Grenzwert ist,
   **dadurch gekennzeichnet, dass**

die Auswerteinheit (30B) Mittel (33) zum fortlaufenden Bestimmen des vorgegebenen Grenzwerts $U_{trigger}$ auf der Grundlage des zeitlichen Verlaufs des Pumpenstroms oder der mit dem Pumpenstrom korrelierenden Größe aufweist,
wobei die Mittel (31) zum Vergleichen des Pumpenstroms oder der mit dem Pumpenstrom korrelierenden Größe derart ausgebildet sind, dass der Pumpenstrom oder die mit dem Pumpenstrom korrelierende Größe mit dem fortlaufend bestimmten Grenzwert zur Erkennung eines periodisch auftretenden Anstiegs des Pumpenstroms oder der mit dem Pumpenstrom korrelierenden Größe verglichen wird.

2. Vorrichtung nach Anspruch 1, **dadurch gekennzeichnet, dass** die Mittel (33) zum fortlaufenden Bestimmen des vorgegebenen Grenzwerts $U_{trigger}$ derart ausgebildet sind, dass der vorgegebene Grenzwert aus einem ersten Wert $U_{min}$ berechnet wird, der für den Pumpenstrom oder die mit dem Pumpenstrom korrelierenden Größe vor dem periodisch auftretenden Anstieg des Pumpenstroms charakteristisch ist, und einem zweiten Wert $U_{max}$ berechnet wird, der für den Pumpenstrom oder die mit dem Pumpenstrom korrelierenden Größe während des periodisch auftretenden Anstiegs des Pumpenstroms oder der mit dem Pumpenstrom korrelierenden Größe charakteristisch ist.

3. Vorrichtung nach Anspruch 2, **dadurch gekennzeichnet, dass** der erste Wert der Minimalwert $U_{min}$ des Pumpenstroms oder der mit dem Pumpenstrom korrelierenden Größe vor dem periodisch auftretenden Anstieg ist, wobei die Mittel (33) zum fortlaufenden Bestimmen des vorgegebenen Grenzwerts derart ausgebildet sind, dass der Pumpenstrom oder die mit dem Pumpenstrom korrelierende Größe fortlaufend auf den Minimalwert überwacht wird.

4. Vorrichtung nach Anspruch 3, **dadurch gekennzeichnet, dass** die Mittel (33) zum fortlaufenden Bestimmen des vorgegebenen Grenzwerts derart ausgebildet sind, dass der mit der Messeinheit (30A) momentan gemessene Wert für den Pumpenstrom mit dem zuvor ermittelten Minimalwert für den Pumpenstrom verglichen wird, wobei der momentan gemessene Wert als neuer Minimalwert angenommen wird, wenn der momentan gemessene Wert kleiner als der zuvor ermittelte Minimalwert ist.

5. Vorrichtung nach einem der Ansprüche 2 bis 4, **dadurch gekennzeichnet, dass** der zweite Wert der Maximalwert $U_{max}$ des Pumpenstroms oder der mit dem Pumpenstrom korrelierenden Größe während des periodisch auftretenden Anstiegs ist, wobei die Mittel (33) zum fortlaufenden Bestimmen des vorgegebenen Grenzwerts derart ausgebildet sind, dass der Pumpenstrom oder die mit dem Pumpenstrom korrelierende Größe fortlaufend auf den Maximalwert überwacht wird.

6. Vorrichtung nach Anspruch 5, **dadurch gekennzeichnet, dass** die Mittel (33) zum fortlaufenden Bestimmen des vorgegebenen Grenzwerts derart ausgebildet sind, dass der mit der Messeinheit (30A) momentan gemessene Wert für den Pumpenstrom mit dem zuvor ermittelten Maximalwert für den Pumpenstrom verglichen wird, wobei der momentan gemessene Wert als neuer Maximalwert angenommen wird, wenn der momentan gemessene Wert größer als der zuvor ermittelte Maximalwert ist.

7. Vorrichtung nach einem der Ansprüche 1 bis 6, **dadurch gekennzeichnet, dass** die Mittel (33) zum fortlaufenden Bestimmen des vorgegebenen Grenzwerts ein Zeitglied (33A) mit einem vorgegebenen Zeitintervall aufweisen, wobei die Mittel (33) zum fortlaufenden Bestimmen des vorgegebenen Grenzwerts derart ausgebildet sind, dass das Zeitglied (33A) gestartet wird, wenn ein Anstieg des Pumpenstroms oder der mit dem Pumpenstrom korrelierenden Größe erkannt wird, wobei innerhalb des vorgegebenen Zeitintervalls die Erzeugung des Steuersignals unterbrochen wird.

8. Vorrichtung nach einem der Ansprüche 5 bis 7, **dadurch gekennzeichnet, dass** die Mittel (33) zum fortlaufenden Bestimmen des vorgegebenen Grenzwerts derart ausgebildet sind, dass der vorgegebene Grenzwert $U_{trigger}$ aus einer mit dem gemessenen Pumpenstrom korrelierenden Größe wie folgt berechnet wird:

$$U_{trigger} = U_{min} + ((U_{max} - U_{min})/2)$$

wobei: $U_{min}$ der Minimalwert für die mit dem Pumpenstrom korrelierende Größe und $U_{max}$ der Maximalwert für die mit dem Pumpenstrom korrelierende Größe ist.

9. Vorrichtung zum Betreiben einer Bilanzkammer einer Bilanziereinrichtung einer extrakorporalen Blutbehandlungsvorrichtung mit einer elektrisch betriebenen Pumpe (7), **dadurch gekennzeichnet, dass** eine Vorrichtung nach einem der Ansprüche 1 bis 8 zur Überwachung der elektrisch betriebenen Pumpe (7) zum Betreiben der Bilanzkammer vorgesehen ist.

10. Vorrichtung zur extrakorporalen Blutbehandlung mit einer Vorrichtung nach einem der Ansprüche 1 bis 9.

11. Verfahren zur Überwachung einer im Flüssigkeitssystem einer extrakorporalen Blutbehandlungsvorrichtung angeordneten elektrisch betriebenen Pumpe mit folgenden Verfahrensschritten:

Messen des Pumpenstroms oder einer mit dem Pumpenstrom korrelierenden Größe,
Auswerten des zeitlichen Verlaufs des Pumpenstroms oder der mit dem Pumpenstrom korrelierende Größe, wobei:

der Pumpenstrom oder die mit dem Pumpenstrom korrelierende Größe mit einem vorgegebenen Grenzwert verglichen wird, und
ein Steuersignal erzeugt wird, wenn der Pumpenstrom oder die mit dem Pumpenstrom korrelierende Größe größer als der vorgegebene Grenzwert ist,
**dadurch gekennzeichnet, dass**
der vorgegebene Grenzwert auf der Grundlage des zeitlichen Verlaufs des Pumpenstroms oder der mit dem Pumpenstrom korrelierenden Größe fortlaufend bestimmt wird,
wobei der Pumpenstrom oder die mit dem Pumpenstrom korrelierende Größe mit dem fortlaufend bestimmten Grenzwert zur Erkennung eines periodisch auftretenden Anstiegs des Pumpenstroms oder der mit dem Pumpenstrom korrelierenden Größe verglichen wird.

12. Verfahren nach Anspruch 11, **dadurch gekennzeichnet, dass** der vorgegebene Grenzwert aus einem ersten Wert berechnet wird, der für den Pumpenstrom oder die mit dem Pumpenstrom korrelierenden Größe vor dem periodisch auftretenden Anstieg des Pumpenstroms charakteristisch ist, und einem zweiten Wert berechnet wird, der für den Pumpenstrom oder die mit dem Pumpenstrom korrelierende Größe während des periodisch auftretenden Anstiegs des Pumpenstroms oder der mit dem Pumpenstrom korrelierenden Größe charakteristisch ist.

13. Verfahren nach Anspruch 12, **dadurch gekennzeichnet, dass** der erste Wert der Minimalwert des Pumpenstroms oder der mit dem Pumpenstrom korrelierenden Größe vor dem periodisch auftretenden Anstieg des Pumpenstroms oder der mit dem Pumpenstrom korrelierenden Größe ist, wobei der Pumpenstrom oder die mit dem Pumpenstrom korrelierende Größe fortlaufend auf den Minimalwert überwacht wird.

14. Verfahren nach Anspruch 13, **dadurch gekennzeichnet, dass** der momentan gemessene Wert für den Pumpenstrom mit dem zuvor ermittelten Minimalwert für den Pumpenstrom verglichen wird, wobei der momentan gemessene Wert als neuer Minimalwert angenommen wird, wenn der momentan gemessene Wert kleiner als der zuvor ermittelte Minimalwert ist.

15. Verfahren nach einem der Ansprüche 12 bis 14, **dadurch gekennzeichnet, dass** der zweite Wert der Maximalwert des Pumpenstroms oder der mit dem Pumpenstrom korrelierenden Größe während des periodisch auftretenden Anstiegs des Pumpenstroms oder der mit dem Pumpenstrom korrelierenden Größe ist, wobei der Pumpenstrom oder die mit dem Pumpenstrom korrelierende Größe fortlaufend auf den Maximalwert überwacht wird.

16. Verfahren nach Anspruch 15, **dadurch gekennzeichnet, dass** der momentan gemessene Wert für den Pumpenstrom oder der mit dem Pumpenstrom korrelierenden Größe mit dem zuvor ermittelten Maximalwert für den Pumpenstrom verglichen wird, wobei der momentan gemessene Wert als neuer Maximalwert angenommen wird, wenn der momentan gemessene Wert größer als der zuvor ermittelte Maximalwert ist.

17. Verfahren nach einem der Ansprüche 11 bis 16, **dadurch gekennzeichnet, dass** ein Zeitglied mit einem vorgegebenen Zeitintervall gestartet wird, wenn ein Anstieg des Pumpenstroms oder der mit dem Pumpenstrom korrelierenden Größe erkannt wird, wobei innerhalb des vorgegebenen Zeitintervalls die Erzeugung des Steuersignals unterbrochen wird.

18. Verfahren nach einem der Ansprüche 15 bis 17, **dadurch gekennzeichnet, dass** der vorgegebene Grenzwert $U_{trigger}$ wie folgt berechnet wird:

$$U_{trigger} = U_{min} + ((U_{max} - U_{min})/2)$$

wobei: $U_{min}$ der Mininalwert für die mit dem Pumpenstrom korrelierende Größe und $U_{max}$ der Maximalwert für die mit dem Pumpenstrom korrelierende Größe ist.

19. Verfahren zum Betreiben einer Bilanzkammer einer Bilanziereinrichtung einer extrakorporalen Blutbehandlungsvorrichtung mit einer elektrisch betriebenen Pumpe, **dadurch gekennzeichnet, dass** die elektrisch betriebene Pumpe mit einem Verfahren nach einem der Ansprüche 11 bis 18 überwacht wird.

**Claims**

1. Device for monitoring an electrically operated pump arranged in the fluid system of an extracorporeal blood treatment device, comprising
a measuring unit (30A) for measuring the pump current or a variable correlated to the pump current;
an evaluation unit (30B) for evaluating the evolution over time in the pump current or in the variable correlated to the pump current, comprising:

means (31) for comparing the pump current or the variable correlated to the pump current with a predetermined threshold,
means (32) for generating a control signal if the pump current or the variable correlated to the pump current is larger than the predetermined threshold,
**characterised in that**
the evaluation unit (30B) comprises means (33) for continuously setting the predetermined threshold $U_{trigger}$ on the basis of the evolution over time in the pump current or in the variable correlated to the pump current,
the means (31) for comparing the pump current or the variable correlated to the pump current being formed such that the pump current or the variable correlated to the pump current is compared with the continuously set predetermined threshold in order to identify a periodic rise in the pump current or in the variable correlated to the pump current.

2. Device according to claim 1, **characterised in that** the means (33) for continuously setting the predetermined threshold $U_{trigger}$ are formed such that the predetermined threshold is calculated from a first value $U_{min}$ that is characteristic of the pump current or the variable correlated to the pump current prior to the periodic rise in the pump current, and a second value $U_{max}$ is calculated that is characteristic of the pump current or the variable correlated to the pump current during the periodic rise in the pump current or in the variable correlated to the pump current.

3. Device according to claim 2, **characterised in that** the first value is the minimum value $U_{min}$ for the pump current or the variable correlated to the pump current prior to the periodic rise, the means (33) for continuously setting the predetermined threshold being formed such that the pump current or the variable correlated to the pump current is continuously monitored at the minimum value.

4. Device according to claim 3, **characterised in that** the means (33) for continuously setting the predetermined threshold are formed such that the present pump current reading taken by the measuring unit (30A) is compared with the previously established minimum value for the pump current, the present reading being adopted as the new minimum value if the present reading is smaller than the previously established minimum value.

5. Device according to any of claims 2 to 4, **characterised in that** the second value is the maximum value $U_{max}$ for the pump current or the variable correlated to the pump current during the periodic rise, the means (33) for continuously setting the predetermined threshold being formed such that the pump current or the variable correlated to the pump current is continuously monitored at the maximum value.

6. Device according to claim 5, **characterised in that** the means (33) for continuously setting the predetermined threshold are formed such that the present pump current reading taken by the measuring unit (30A) is compared with the previously established maximum pump current value, the present reading being adopted as the new maximum value if the present reading is larger than the previously established maximum value.

7. Device according to any of claims 1 to 6, **characterised in that** the means (33) for continuously setting the predetermined threshold comprise a time function element (33A) having a predetermined time interval, the means (33) for continuously setting the predetermined threshold being formed such that the time function element (33A) is started if a rise in the pump current or in the variable correlated to the pump current is identified, generation of the control signal being interrupted within the predetermined time interval.

8. Device according to any of claims 5 to 7, **characterised in that** the means (33) for continuously setting the predetermined threshold are formed such that the predetermined threshold $U_{trigger}$ is calculated from a variable correlated to the measured pump current as follows:

$$U_{trigger} = U_{min} + ((U_{max} - U_{min})/2)$$

$U_{min}$ being the minimum value for the variable correlated to the pump current and $U_{max}$ being the maximum value for the variable correlated to the pump current.

9. Device for operating a balancing chamber of a balancing apparatus for an extracorporeal blood treatment device having an electrically operated pump (7), **characterised in that** a device according to any of claims 1 to 8 is provided for monitoring the electrically operated pump (7) for operating the balancing chamber.

10. Device for extracorporeal blood treatment having a device according to any of claims 1 to 9.

11. Method for monitoring an electrically operated pump arranged in the fluid system of an extracorporeal blood treatment device, comprising the following method steps:

measuring the pump current or a variable correlated to the pump current,
evaluating the evolution over time in the pump current or in the variable correlated to the pump current,
the pump current or the variable correlated to the pump current being compared with a predetermined threshold, and
a control signal being generated if the pump current or the variable correlated to the pump current is larger than the predetermined threshold,
**characterised in that**
the predetermined threshold is continuously set on the basis of the evolution over time in the pump current or in the variable correlated to the pump current,
the pump current or the variable correlated to the pump current being compared with the continuously set threshold in order to identify a periodic rise in the pump current or in the variable correlated to the pump current.

12. Method according to claim 11, **characterised in that** the predetermined threshold is calculated from a first value that is characteristic of the pump current or the variable correlated to the pump current prior to the periodic rise in the pump current, and is calculated from a second value that is characteristic of the pump current or the variable correlated to the pump current during the periodic rise in the pump current or in the variable correlated to the pump current.

13. Method according to claim 12, **characterised in that** the first value is the minimum value for the pump current or the variable correlated to the pump current prior to the periodic rise in the pump current or in the variable correlated to the pump current, the pump current or the variable correlated to the pump current being continuously monitored at the minimum value.

14. Method according to claim 13, **characterised in that** the present pump current reading is compared with the previously established minimum pump current value, the present reading being adopted as the new minimum value if the present reading is smaller than the previously established minimum value.

15. Method according to any of claims 12 to 14, **characterised in that** the second value is the maximum value for the pump current or the variable correlated to the pump current during the periodic rise in the pump current or in the variable correlated to the pump current, the pump current or the variable correlated to the pump current being continuously monitored at the maximum value.

**16.** Method according to claim 15, **characterised in that** the present reading for the pump current or the variable correlated to the pump current is compared with the previously established maximum pump current value, the present reading being adopted as the new maximum value if the present reading is greater than the previously established maximum value.

**17.** Method according to any of claims 11 to 16, **characterised in that** a time function element is started at a predetermined time interval if a rise in the pump current or in the variable correlated to the pump current is identified, generation of the control signal being interrupted within the predetermined time interval.

**18.** Method according to any of claims 15 to 17, **characterised in that** the predetermined threshold $U_{trigger}$ is calculated as follows:

$$U_{trigger} = U_{min} ((U_{max} - U_{min})/^2)$$

$U_{min}$ being the minimum value for the variable correlated to the pump current and $U_{max}$ being the maximum value for the variable correlated to the pump current.

**19.** Method for operating a balancing chamber of a balancing apparatus of an extracorporeal blood treatment device having an electrically operated pump, **characterised in that** the electrically operated pump is monitored using a method according to any of claims 11 to 18.

**Revendications**

**1.** Dispositif de surveillance d'une pompe à fonctionnement électrique disposée dans le système de liquide d'un dispositif de traitement extracorporel du sang, comportant une unité de mesure (30A) pour mesurer le flux de pompage ou une grandeur correspondant au courant de pompage,
une unité d'analyse (30B) pour analyser l'évolution temporelle du courant de pompage ou de la grandeur correspondant au courant de pompage, qui présente ;
des moyens (31) de comparaison du courant de pompage ou la grandeur correspondant au courant de pompage à une valeur seuil prédéterminée,
des moyens (32) de génération d'un signal de commande lorsque le courant de pompage ou la grandeur correspondant au courant de pompage est supérieur(e) à la valeur seuil prédéterminée,
**caractérisé en ce que**
l'unité d'analyse (30B) présente des moyens (33) de détermination en continu de la valeur seuil prédéterminée $U_{trigger}$ sur la base de l'évolution temporelle du courant de pompage ou de la grandeur correspondant au courant de pompage,
dans lequel les moyens (31) de comparaison du courant de pompage ou de la grandeur correspondant au courant de pompage sont conçus de telle sorte que le courant de pompage ou la grandeur correspondant au courant de pompage est comparé(e) à la valeur seuil déterminée en continu pour la reconnaissance d'une augmentation survenant de façon périodique du courant de pompage ou de la grandeur correspondant au courant de pompage.

**2.** Dispositif selon la revendication 1, **caractérisé en ce que** les moyens (33) pour la détermination en continu de la valeur seuil prédéterminée $U_{trigger}$ sont conçus de telle sorte que la valeur seuil prédéterminée est calculée à partir d'une première valeur $U_{min}$ qui est caractéristique du courant de pompage ou qui est caractéristique de la grandeur correspondant au courant de pompage avant l'augmentation survenant périodiquement du courant de pompage et à partir d'une deuxième valeur $U_{max}$ qui est caractéristique du courant de pompage ou qui est caractéristique de la grandeur correspondant au courant de pompage pendant l'augmentation survenant périodiquement du courant de pompage ou de la grandeur correspondant au courant de pompage.

**3.** Dispositif selon la revendication 2, **caractérisé en ce que** la première valeur est la valeur minimale $U_{min}$ du courant de pompage ou la grandeur correspondant au courant de pompage avant l'augmentation survenant périodiquement, dans lequel les moyens (33) de détermination continue de la valeur seuil prédéterminée sont conçus de telle sorte que la valeur minimale du courant de pompage ou de la grandeur correspondant au courant de pompage est surveillée en continu.

**4.** Dispositif selon la revendication 3, **caractérisé en ce que** les moyens (33) de détermination continue de la valeur seuil prédéterminée sont conçus de telle sorte que la valeur mesurée de façon instantanée avec l'unité de mesure (30A) du courant de pompage est comparée à la valeur minimale déterminée précédemment du courant de pompage, dans lequel on admet que la valeur mesurée de façon instantanée est une nouvelle valeur minimale lorsque la valeur mesurée de façon instantanée est inférieure à la valeur minimale déterminée précédemment.

**5.** Dispositif selon l'une des revendications 2 à 4, **caractérisé en ce que** la deuxième valeur est la valeur maximale $U_{max}$ du courant de pompage ou de la grandeur correspondant au courant de pompage pendant l'augmentation survenant de façon périodique, dans lequel les moyens (33) de détermination continue de la valeur seuil prédéterminée sont conçus de telle sorte que la valeur maximale du courant de pompage ou de la grandeur correspondant au courant de pompage est surveillée en continu.

**6.** Dispositif selon la revendication 5, **caractérisé en ce que** les moyens (33) de détermination continue de la valeur seuil prédéterminée sont conçus de telle sorte que la valeur mesurée de façon instantanée avec l'unité de mesure (30A) du courant de pompage est comparée à la valeur maximale déterminée précédemment du courant de pompage, dans lequel on admet que la valeur mesurée de façon instantanée est une nouvelle valeur maximale lorsque la valeur mesurée de façon instantanée est supérieure à la valeur maximale déterminée précédemment.

**7.** Dispositif selon l'une des revendications 1 à 6, **caractérisé en ce que** les moyens (33) de détermination continue de la valeur seuil prédéterminée présentent un élément de temporisation (33A) présentant un intervalle temporel prédéterminé, dans lequel les moyens (33) de détermination continue de la valeur seuil prédéterminée sont conçus de telle sorte que l'élément de temporisation (33A) est lancé lorsqu'une augmentation du courant de pompage ou de la grandeur correspondant au courant de pompage est reconnue, dans lequel la génération du signal de commande est interrompue dans l'intervalle temporel prédéterminé.

**8.** Dispositif selon l'une des revendications 5 à 7, **caractérisé en ce que** les moyens (33) de détermination continue de la valeur seuil prédéterminée sont conçus de telle sorte que la valeur seuil prédéterminée $U_{trigger}$ est calculée à partir de la grandeur correspondant au courant de pompage mesurée, comme suit :

$$U_{trigger} = U_{min} + ((U_{max} - U_{min})/2)$$

dans laquelle : $U_{min}$ est la valeur minimale de la grandeur correspondant au courant de pompage et $U_{max}$ est la valeur maximale de la grandeur correspondant au courant de pompage.

**9.** Dispositif d'actionnement d'une chambre d'équilibrage d'un système d'équilibrage d'un dispositif de traitement extracorporel du sang comportant une pompe à fonctionnement électrique (7), **caractérisé en ce qu'**un dispositif selon l'une des revendications 1 à 8 est prévu pour la surveillance de la pompe à fonctionnement électrique (7) pour actionner la chambre d'équilibrage.

**10.** Dispositif de traitement extracorporel du sang comportant un dispositif selon l'une des revendications 1 à 9.

**11.** Procédé de surveillance d'une pompe à fonctionnement électrique disposée dans le système de liquide d'un dispositif de traitement extracorporel du sang, comportant les étapes du procédé suivantes :

mesure du courant de pompage ou d'une grandeur correspondant au courant de pompage, analyse de l'évolution temporelle du courant de pompage ou de la grandeur correspondant au courant de pompage, dans lequel :

le courant de pompage ou la grandeur correspondant au courant de pompage est comparé(e) à une valeur seuil prédéterminée, et
un signal de commande est généré lorsque le courant de pompage ou la grandeur correspondant au courant de pompage est supérieur(e) à la valeur seuil prédéterminée,
**caractérisé en ce que**
la valeur seuil prédéterminée est déterminée en continu sur la base de l'évolution temporelle du courant de pompage ou de la grandeur correspondant au courant de pompage,
dans lequel le courant de pompage ou la grandeur correspondant au courant de pompage est comparée à la valeur seuil déterminée en continu pour la reconnaissance d'une augmentation survenant périodiquement du courant de pompage ou de la grandeur correspondant au courant de pompage.

**12.** Procédé selon la revendication 11, **caractérisé en ce que** la valeur seuil prédéterminée est calculée à partir d'une première valeur qui est caractéristique du courant de pompage ou de la grandeur correspondant au courant de pompage avant l'augmentation survenant périodiquement du courant de pompage et à partir d'une deuxième valeur qui est caractéristique du courant de pompage ou de la grandeur correspondant au courant de pompage pendant l'augmentation survenant périodiquement du courant de pompage ou de la grandeur correspondant au courant de pompage.

**13.** Procédé selon la revendication 12, **caractérisé en ce que** la première valeur est la valeur minimale du courant de pompage ou de la grandeur correspondant au courant de pompage avant l'augmentation survenant périodiquement du courant de pompage ou de la grandeur correspondant au courant de pompage, dans lequel la valeur minimale du courant de pompage ou de la grandeur correspondant au courant de pompage est surveillée en continu.

**14.** Procédé selon la revendication 13, **caractérisé en ce que** la valeur mesurée de façon instantanée du courant de pompage est comparée à la valeur minimale déterminée précédemment du courant de pompage, dans lequel on admet que la valeur mesurée de façon instantanée est une nouvelle valeur minimale lorsque la valeur mesurée de façon instantanée est inférieure à la valeur minimale déterminée précédemment.

**15.** Procédé selon l'une des revendications 12 à 14, **caractérisé en ce que** la deuxième valeur est la valeur maximale du courant de pompage ou de la grandeur correspondant au courant de pompage pendant l'augmentation survenant périodiquement du courant de pompage ou de la grandeur correspondant au courant de pompage, dans lequel la valeur maximale du courant de pompage ou de la grandeur correspondant au courant de pompage est surveillée en continu.

**16.** Procédé selon la revendication 15, **caractérisé en ce que** la valeur mesurée de façon instantanée du courant de pompage ou de la grandeur correspondant au courant de pompage est comparée à la valeur maximale déterminée précédemment du courant de pompage, dans lequel on admet que la valeur mesurée de façon instantanée est une nouvelle valeur maximale lorsque la valeur mesurée de façon instantanée est supérieure à la valeur maximale déterminée précédemment.

**17.** Procédé selon l'une des revendications 11 à 16, **caractérisé en ce qu'**un élément de temporisation comportant un intervalle temporel prédéterminé est lancé, lorsqu'une augmentation du courant de pompage ou de la grandeur correspondant au courant de pompage est reconnu(e), dans lequel dans l'intervalle temporel prédéterminé, la génération du signal de commande est interrompue.

**18.** Procédé selon l'une des revendications 15 à 17, **caractérisé en ce que** la valeur seuil prédéterminée $U_{trigger}$ est calculée comme suit :

$$U_{trigger} = U_{min} + ((U_{max} - U_{min})/2)$$

dans laquelle : $U_{min}$ est la valeur minimale de la grandeur correspondant au courant de pompage et $U_{max}$ est la valeur maximale de la grandeur correspondant au courant de pompage.

**19.** Procédé de surveillance d'une chambre d'équilibrage d'un système d'équilibrage d'un dispositif de traitement extracorporel du sang comportant une pompe à fonctionnement électrique, **caractérisé en ce que** la pompe à fonctionnement électrique est surveillée avec un procédé selon l'une des revendications 11 à 18.

Fig. 1

EP 2 509 653 B1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6042784 A
- DE 19830928

- DE 2838414